# EUROPEAN PATENT SPECIFICATION

(11) **EP 3 956 081 B1**
(45) Date of publication and mention of the grant of the patent: **19.06.2024**
(21) Application number: 20791637.0
(22) Date of filing: 17.04.2020
(51) Int. Cl.: B09C 1/06, B09C 1/10, C02F 3/34, C12N 1/20, C12R 1/01, C02F 101/32, C02F 103/06

(54) **METHOD FOR BIOREMEDIATION OF SOIL**
VERFAHREN ZUR BIOSANIERUNG VON BODEN
PROCÉDÉ DE BIORESTAURATION D'UN SOL

(30) Priority: 17.04.2019 US 201962835148 P
(43) Date of publication of application: 23.02.2022
(73) Proprietor: The Trustees of Columbia University in the City of New York, New York, NY 10027 (US)
(72) Inventor: SAMBROTTO, Raymond, NY 10964 (US)
(74) Representative: FRKelly
(86) International application number: PCT/US2020/028642
(87) International publication number: WO 2020/214890

(56) References cited:
- US-A1- 2005 208 642
- US-A1- 2008 210 630
- US-A1- 2013 295 650
- US-A1- 2013 295 650
- HOSHINA S ET AL: "BIODEGRADATION PATHWAY OF DIOXINS BY NOVEL RAPID GROWING THERMOPHILE", ORGANOHALOGEN COMPOUNDS, XX, XX, vol. 45, 1 January 2000 (2000-01-01), pages 384-386, XP001077834,
- XIAO et al.: "Determination of Vitamins B2, B3, B6 and B7 in Corn Steep Liquor by NIR and PLSR", Trans. Tianjin Univ., vol. 18, no. 5, 5 October 2012 (2012-10-05), pages 372-377, XP035121296,
- TAKAHASHI et al.: "Feasibility Study on Bio-degradation System for Polychlorinated Dioxins In Contaminated Soil and Sediments", Organohalogen Compounds, vol. 68, 2006, pages 2361-2364, XP055750142,
- RAMOS et al.: "Assessment of microbial communities associated with fermentative- methanogenic biodegradation of aromatic hydrocarbons in groundwater contaminated with a biodiesel blend (B20)", Biodegradation, vol. 25, no. 5, 19 April 2014 (2014-04-19) , pages 681-691, XP055750144,
- DONY et al.: "Comparison of the sensitivity of Pseudomonas aeruginosa to disinfectants according to the growth conditions", , vol. 19, no. 2, April 1984 (1984-04), pages 239-242, XP023844631, DOI: 10.1016/0378-5173(84)90167-4

## Description

### FIELD OF THE INVENTION

The present invention relates to the field of environmental restoration techniques for soils and sediments contaminated with toxic organic pollutants and, more specifically methods for enhanced remediation of contaminated materials using a *Geobacillus sp.* bacterial strain or an enhanced variant thereof.

### BACKGROUND OF THE INVENTION

Human industrial activities generate a large amount and diversity of pollutant and toxic compounds. Among these are the carcinogenic polycyclic aromatic hydrocarbons (PAHs), that are extremely damaging to the environment and human health. The remediation of soils and sediments contaminated with PAHs typically depend on landfills or incineration. Bioremediation offers a less expensive, eco-friendly alternative that uses microorganisms to degrade these toxic contaminants in matrixes such as soil, sediments or water.

The level of success of bioremediation efforts is largely dependent on the type of microbes used and their capability to destroy or degrade toxic compounds. However, current bioremediation approaches have a limited range of treatable pollutants. Furthermore, the degradation products themselves can be toxic and require additional degradation. Other limitations of bioremediation are (i) the complex scientific knowledge required to customize an appropriate treatment plan, (ii) the difficulty of maintaining aerobic conditions to optimize degradation for those strains that require oxygen and (iii) the bioavailability of contaminants.

It is with respect to these and other considerations that the disclosure is presented.

*Geobacillus midousuji* bacteria produce a variety of chemically and biologically active molecules that can be used to replace harsh reactants in chemical synthesis, green chemistry or bioremediation applications. These compounds include surfactants and proteases, as well as other enzymes that can be used for enantiospecific reactions or the suppression of reactive oxygen species in chemical or biological systems. The bacteria can be used in remediation of chemical waste, including poly-chlorinated biphenyls (PCBs), poly-aromatic hydrocarbons (PAHs), and perfluorochemicals. Reactions can be carried out under both aerobic and anaerobic conditions across a wide range of temperatures, which has the potential to significantly reduce the cost of current remediation.

We have identified various strains of *Geobacillus midousuji* bacteria that can be used to decontaminate soil and sediments or other contaminated matrixes, e.g., water supplies, of polycyclic aromatic hydrocarbons (PAH) and more specifically, benzo[a]pyrene (BaP). Because decontamination can occur under both aerobic or anaerobic conditions, the costs of remediation are lower. This work also expands the range of pollutants treatable by bioremediation with *Geobacillus midousuji.*

Document US 2013/295650 A1 discloses a method for bioremediation of soil, sediment or wastewater containing benzo[a]pyrene (BaP) using crude enzyme extract of Geobacillus midousuii.

### SUMMARY

The present invention provides for a method for bioremediation of soil, sediment or wastewater (e.g., groundwater) containing benzo[a]pyrene (BaP) according to claim 1. The method comprises the steps of: (a) administering live

*Geobacillus midousuji,* or a mutant derived therefrom (e.g., having degrading activity of the *Geobacillus midousuji*), to the soil, sediment or wastewater to be bioremediated; and, (b) incubating the *Geobacillus midousuji* in the soil, sediment or wastewater at a temperature ranging from about 40 to about 70°C and a humidity ranging from about 80% to about 100% for a period of time ranging from about 1 hour to about 20 days.

The *Geobacillus midousuji* may be strain SH2B (American Type Culture Collection (ATCC) No. 55926) or a mutant derived therefrom (e.g., retaining the degrading activity thereof), strain SH2A (ATCC No. 202050) or a mutant derived therefrom (e.g., retaining the degrading activity thereof), or mixtures thereof.

In certain embodiments, the BaP concentration in the soil, sediment or wastewater after incubation is less than about 100 parts per million (PPM), less than about 50 PPM, or less than about 20 PPM.

The *Geobacillus midousuji* to the soil, sediment or wastewater ratio may range from about 0.01% to about 1% w/w, from about 0.01% to about 0.1% w/w, from about 0.08% w/w to about 0.15% w/w, or about 0.1% w/w.

In certain embodiments, the *Geobacillus midousuji* is activated into log phase growth before incubation with the soil, sediment or wastewater by incubation of the *Geobacillus midousuji* under an aerobic condition at a temperature ranging from about 60° to about 65° C in a medium comprising B-complex vitamin and amino-N with trace metals.

The method may further comprise the step of rotating the soil, sediment or wastewater at least about 1 to about 5 times per day during incubation with the *Geobacillus midousuji.*

The incubation of the *Geobacillus midousuji* in the soil, sediment or wastewater may be done under aerobic conditions. The incubation of the *Geobacillus midousuji* in the soil, sediment or wastewater may be done under anaerobic conditions.

The method may further comprise purifying or harvesting at least one protein product and/or at least one surfactant product.

The present disclosure provides for a kit comprising: (i) *Geobacillus midousuji* or a mutant derived therefrom; (ii) printed matter with instructions for activating the *Geobacillus midousuji* into log phase growth before incubation with soil, sediment or wastewater by incubation of the *Geobacillus midousuji* under aerobic conditions at a temperature ranging from about 60° to about 65° C in a medium comprising B-complex vitamin and amino-N with trace metals; and, instructions for use of the *Geobacillus midousuji* in the soil, sediment or wastewater, wherein the soil, sediment or wastewater is maintained at a temperature ranging from about 40°C to about 70°C and a humidity ranging from about 90% to about 100% for a period of time ranging from about 1 hour to about 20 days.

The *Geobacillus midousuji* may be strain SH2B (American Type Culture Collection (ATCC) No. 55926) or a mutant derived therefrom (e.g., retaining the degrading activity thereof), strain SH2A (ATCC No. 202050) or a mutant derived therefrom (e.g., retaining the degrading activity thereof), or mixtures thereof.

According to the present invention, a method for bioremediation of soil containing benzo[a]pyrene (BaP) is provided. The method comprises the steps of administering to the soil to be bioremediated, *Geobacillus midousuji,* designated ATCC55926 strain SH2B, an enhanced bacterial strain of ATCC202050, ATCC55926, or mixtures thereof, at a ratio ranging from about 0.01% to about 1% (w/w of wet weight of the *Geobacillus midousuji* to weight of the soil). The method also includes the step of incubating the *Geobacillus midousuji* in the soil, wherein the soil is maintained at a temperature ranging from about 40 to 70°C and a humidity ranging from about 80% to about 100% for a period of time ranging from about 1 hour to about 480 hours.

The present disclosure also provides a kit that comprises: *Geobacillus midousuji,* designated ATCC55926 strain SH2B, an enhanced bacterial strain of ATCC202050, ATCC55926, or mixtures thereof at a ratio ranging from about 0.01% to about 1% (w/w of wet weight of the Geobacillus midousuji to weight of the soil). The kit also includes printed matter with instructions for activating the *Geobacillus midousuji* into log phase growth before incubation with the soil by incubation of the *Geobacillus midousuji* under aerobic conditions at a temperature ranging from about 60° to about 65° C in a medium comprising B-complex vitamin and amino-N with trace metals. In addition, the kit includes instructions for use of the *Geobacillus midousuji* in the soil, wherein the soil is maintained at a temperature ranging from about 40 to 70°C and a humidity ranging from about 90% to about 100% for a period of time ranging from about 1 hour to about 180 hours. In another embodiment, the instructions state that the *Geobacillus* strain can be added at ambient temperature, ranging from about 15° C to about 35°C.

These and other aspects, features, and advantages can be appreciated from the accompanying description of certain embodiments of the invention or disclosure and the accompanying drawing figures and claims.

### BRIEF DESCRIPTION OF THE DRAWINGS

FIG. 1 is a flow diagram illustrating an exemplary method for bioremediation of contaminated matter in accordance with one or more embodiments of the invention;
FIG. 2 is a block diagram illustrating a side-view of an exemplary system for bioremediation of contaminated matter according to an embodiment of the present invention;
FIG. 3 is a chart plotting peak height vs. retention time results of degradation experiments with soils containing Benzo[a]pyrene (BaP) and PCBs in accordance with one or more embodiments of the invention;
FIG. 4 is an image illustrating the results of two-dimensional gel electrophoresis of supernatants from a bacterial broth prepared in accordance with one or more embodiments of the invention;
FIG. 5A includes a chart illustrating the results of Matrix-Assisted Laser Desorption/ Ionization - Time of Flight analysis of the 54 kDa protein bands prepared in accordance with one or more embodiments of the invention;
FIG. 5B includes a chart illustrating the results of Matrix-Assisted Laser Desorption/ Ionization - Time of Flight analysis of the 116 kDa protein bands prepared in accordance with one or more embodiments of the invention;
FIG. 6 is a chart plotting optical density vs. incubation time graphically illustrating the enhancement of strain cell yield by optimizing growth media from Trypticase Soy Broth to a modified Terrific Broth prepared in accordance with one or more embodiments of the invention;
FIG. 7 is a graphical representation of the molecular structure of examples of oxidative biotransformation reactions for monooxygenases and examples of oxidative biotransformation reactions for dioxygenases and perioxiases; and

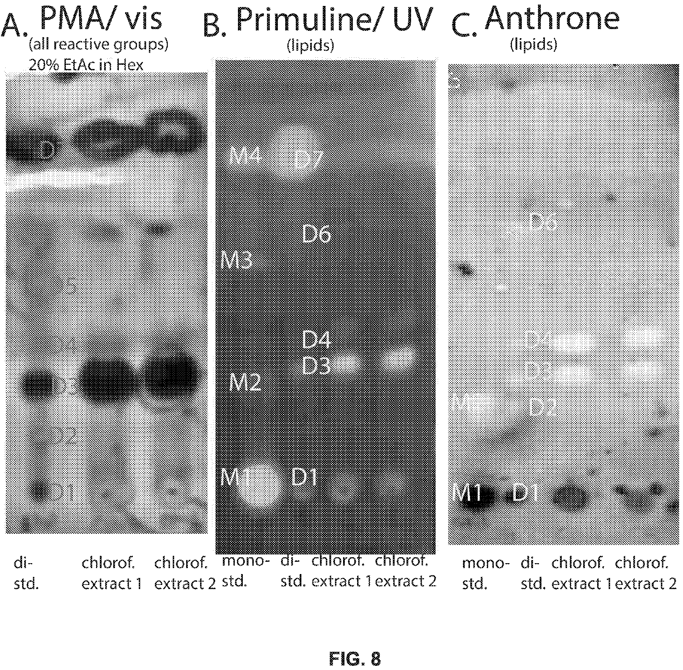

FIG. 8 contains photographs of 3 thin-layer chromatography (TLC) plates showing the analysis of extracts of the spent media from the Geobacillus SH2B strain. Plate A was stained with Phosphomolybdic Acid (PMA) and shows the presence of bands in the samples in lanes 2 and 3 that match the D3 and D4 bands from the di-rhamnolipid standard in lane 1. Plate B was stained with primuline and imaged under UV light and shows the presence of bands in the samples in lanes 3 and 4 that match the D3 and D4 bands from the di-rhamnolipid standard in lane 2. Plate C was stained with anthrone and shows the presence of bands in the samples in lanes 3 and 4 that match the D3 and D4 bands from the di-rhamnolipid standard in lane 2.

### DETAILED DESCRIPTION

By way of overview and introduction, systems of the present disclosure and methods of the present invention are directed to performing bioremediation using a *Geobacillus sp.* bacterial strain, for example, *Geobacillus midousuji* bacterial strain SH2B (ATCC Accession No. 55926) and/or strain SH2A (ATCC No. 202050).

*Geobacillus midousuji* bacteria produce a variety of chemically and biologically active molecules that can be used to replace harsh reactants in chemical synthesis, green chemistry or bioremediation applications. These compounds include surfactants and proteases, as well as other enzymes that can be used for enantiospecific reactions or the suppression of reactive oxygen species in chemical or biological systems. The bacteria can be used in remediation of chemical waste, including poly-chlorinated biphenyls (PCBs), poly-aromatic hydrocarbons (PAHs), and perfluorochemicals (e.g., perfluorooctanoic acid or PFOA). Reactions can be carried out under either aerobic or anaerobic conditions across a wide range of temperatures, which has the potential to significantly reduce the cost of current remediation.

According to one or more embodiments, various strains of *Geobacillus midousuji* bacteria can be used to decontaminate soil and sediments or other contaminated matrixes, e.g., water supplies, of benzo[a]pyrene (BaP). Because decontamination can occur under either aerobic or anaerobic conditions, the costs of remediation are lower. The present disclosure expands the range of pollutants treatable by bioremediation with *Geobacillus midousuji.* The present disclosure achieves various beneficial results including, *inter alia*: extending the applications of *Geobacillus midousuji* to enantiospecific reactions, surfactants and proteases, and the suppression of reactive oxygen species in chemical and biological systems; effectively remediating chemical waste, including polychlorinated biphenyls, poly-aromatic hydrocarbons, and perfluorochemicals; effectively remediating chemical waste in either aerobic or anaerobic conditions across a range of temperatures (40-70°C); being usable in packed-bed columns to remediate the aqueous phase of contaminants in groundwater; and reducing remediation costs.

In the present method, the bacteria *Geobacillus midousuji* may be incubated with the soil, sediment or waste (e.g., wastewater) under oxygen or nitrate (NO₃⁻).

Polycyclic aromatic hydrocarbons (PAH) are a class of environmental pollutants that include carcinogenic forms. They are produced by the incomplete combustion of organic material and in particular, the combustion of petroleum products.

Among the PAHs, benzo[a]pyrene (BaP) is a potent carcinogen and is found in high concentrations in cigarette smoke, processed meat, as well as polluted water, soil, and air. https://pubchem.ncbi.nlm.nih.gov/compound/benzo_a_pyrene, retrieved 12/5/18. The systems and methods disclosed herein, which use these bacterial strains, are capable of effectively degrading BaP, one of the more toxic PAH pollutants. Furthermore, the bacteria used herein are capable of efficiently degrading other ring-based organic contaminants that are also harmful to human health. PAHs are a large group of organic compounds with two or more fused aromatic rings. They have a relatively low solubility in water but are highly lipophilic. Eight PAHs (Car-PAHs) typically considered as possible carcinogens are: benzo(a)anthracene, chrysene, benzo(b) fluoranthene, benzo(k)fluoranthene, benzo(a)pyrene (BaP), dibenzo(a,h)anthracene, indeno(1,2,3-cd)pyrene and benzo (g,h,i) perylene. The US Environmental Protection Agency (EPA) has promulgated 16 unsubstituted PAHs (EPA-PAH) as priority pollutants. Srogi Monitoring of environmental exposure to polycyclic hydrocarbons: a review. Environ Chem Lett 5:169-195 (2007).

Furthermore, the bacteria described herein can be used to treat: PCBs, Dioxins and chlorinated compounds in soil and sediments; dioxane in groundwater. Furthermore, the bacteria is capable of efficiently remediating a growing range of chlorinated solvents and other recalcitrant compounds including: Dioxins and Furans (with tests showing reduction in dioxin levels by 60% in 6 days based on shake flasks and bench scale experiments); PCBs (with tests showing reduction in PCB levels by 50% in 6 days based on lab and scale up experiments); PAHs (with tests showing rapid reduction of PAHs including BaP reduced by 95% in 48 hrs.); BTEX (with tests showing decrease in the concentration of the BTEX components (Benzene, Toluene, Ethylbenzene and Xylene)); and chlorinated solvents (RCRA - US Federal Resource Conservation and Recovery Act, https://www.epa.gov/rcra, retrieved 4-15-20).

*Geobacillus midousuji* (e.g., strain SH2B, ATCC No. 55926; and/or strain SH2A, ATCC No. 202050) are capable of effectively degrading BaP in an environmental situation with contaminated matter such as soil, sediments and water. The term "bacteria" is used throughout the text to refer to *Geobacillus midousuji,* as well as more generally, any other bacterial species that are found to have significant contaminant degrading capabilities. Examples of *Geobacillus midousuji* include strain SH2B (ATCC Accession No. 55926), and/or strain SH2A (ATCC Accession No. 202050). In addition, during growth stages, *Geobacillus midousuji* ATCC Accession No. 55926 strain SH2B and/or Accession NO. 202050 strain SH2A produces a high-yield protein pool containing various enzymes such as dioxygenase and aconitase which can be used in a cell-free system for decontamination. The comparatively high expression of dioxygenase allows for efficient degradation of ring-based organic contaminants. As such, embodiments of the invention can provide both an aerobic and anaerobic solution for treating pollutants through bioremediation. The methods and systems provided herein can reduce remediation costs, reduce energy costs, expand the breadth of pollutant targets, offer a cell-free degradation system, and can be used in a kit.

To the extent certain exemplary embodiments and tests are shown and described as being based mainly on specific strains derived from *Geobacillus midousuji,* for instance, ATCC Accession No. 55926 strain SH2B, the invention is not necessarily so limited. For example, beneficial results are suggested for configurations using ATCC Accession No. 202050 strain SH2A. Discussion of *Geobacillus midousuji* and exemplary approaches for degrading matter with microorganisms ATCC 55926 or 202050 are shown and described in U.S. Patent No. 6,420,165 and US Patent No. 6,190,903. The Geobacillus strains referred to herein deposit details are: (i) bacterium strain, *Bacillus midousuji* SH2A deposited on Jan. 21, 1997 with the American Type Culture Collection (ATCC), 12301 Parklawn Drive, Rockville, Md. 20852, U.S.A., under the provisions of the Budapest Treaty for The International Recognition Of The Deposit Of Microorganisms For The Purposes Of Patent Procedure. Bacterium strain SH2A has been accorded ATCC Accession Number 55926; and, (ii) bacterium strain, *Bacillus midousuji* SH2B deposited on Oct. 24, 1997 which the American Type Culture Collection (ATCC), 12301 Parklawn Drive, Rockville, Md. 20852, U.S.A., under the provisions of the Budapest Treaty for The International Recognition of The Deposit of Microorganisms For The Purposes of Patent Procedure. Bacterium strain SH2B has been accorded ATCC Accession No. 202050.

The organic material degraded by the bacterial strains above include, but should not be limited to, plastics, specifically polyethylene. In one specific embodiment, the polyethylene may be irradiated prior to treatment with the bacterial strains to facilitate the degradative process. Specifically, in such an embodiment, the polyethylene may be irradiated with ultra-violet light.

The organic material degraded by the bacterial strains above may also comprise a protein, specifically waste products of households and such industries as food-processing, agriculture, dairy or fisheries. Specific examples include, but are not limited to, wood pulp, paper products, shellfish, coffee bean dregs, tuna-fish heads, squids and other by-products of these industries. Further kitchen waste may also be degraded by these bacteria. Kitchen waste may include, but are not limited to, paper products, shellfish, coffee bean dregs, tuna-fish heads, squids and other by-products found in kitchen bins, trash dumps and other consumer-based waste.

This disclosure further provides wherein organic material comprising a sugar is degraded by the methods described above. Specifically, such sugars include, but are not limited to, mannose, maltose, trehalose, fructose and raffinose. Many of these sugars may be found in kitchen wastes and are by-products of industries in food-processing and agriculture, e.g. fruits.

The organic material also includes amino acid-based compounds. These compounds may be proteins, polypeptides, peptides, naturally occurring or synthetic. The organic material may also include a nucleic acid molecule, specifically deoxyribonucleic acid molecules. DNAase activity has been described in these bacterial strains. U.S. Patent No. 6,420,165.

The present methods and compositions may be used to treat a waste. The waste may be an industrial waste, an agricultural waste, a human municipal waste, fertilizers, domestic sewage, and industrial effluents. The waste may be manufactured gas plant waste, soot, or used petroleum products. The waste may be produced from incomplete combustion of organic matter.

The present methods may produce at least one protein product which may be chemically and/or biologically active. such as enzymes, protease, 1-pyrroline-carboxylate dehydrogenase (P5C), aconitate hydratase (aconitase), a dioxygenase. The protein product(s) may be used in an enantiospecific reaction. The protein product(s) may be an enantiospecific enzyme, a surfactant, and/or an agent for suppressing reactive oxygen species in a chemical and/or biological system.

### Exemplary bioremediation method:

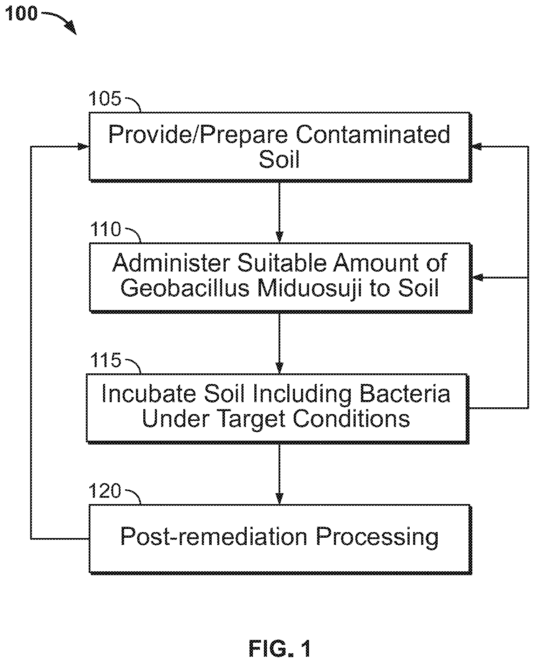

FIG. 1 is a flow diagram illustrating an exemplary method 100 for bioremediation of contaminated matter in accordance with one or more embodiments of the invention. Steps of the method 100 can be performed *in situ,* i.e., without removing the material from its original position, or *ex-situ*, after removal of the soil, sediment, water, etc., from its original location or site, or a combination of either of the foregoing.

*In-situ* bioremediation involves remediation of soils in the ground without removal of the soil to a different location. The exemplary method 100 can also be performed *ex-situ*, for instance, near the original location of the contaminated material, or at a designated soil remediation site some distance away. *Ex-situ* bioremediation is a biological process in which excavated soil is placed in a lined above-ground treatment area. The development of the microbial culture within the remediation zone can be in the presence of oxygen, i.e., under aerobic conditions, or without oxygen, i.e., under anaerobic conditions. The incubation conditions can be dependent on the nature of the contaminants that require degradation and their environmental context. As noted, *Geobacillus midousuji* ATCC 55926 strain SH2B can provide for both an aerobic or anaerobic solution for treating pollutants, including, but not limited to BaP. The preferred embodiment for most contaminants is to carry out the microbial incubation under aerobic conditions because the rates of contaminant degradation are typically greater.

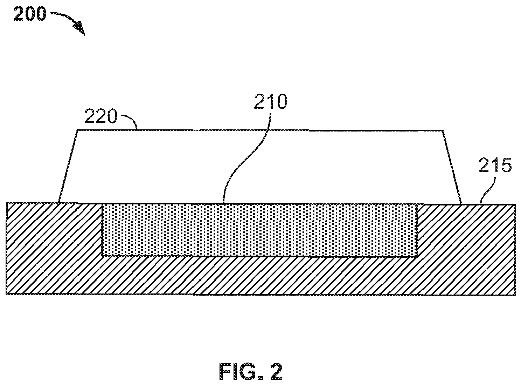

At step 105, a volume or weight of the contaminated soil requiring remediation is provided. For the purpose of illustration, method 100 is described in the context of remediating soil contaminated with BaP *in situ.* FIG. 2 further illustrates the exemplary system 200 in which the exemplary bioremediation method 100 is implemented in accordance with one or more embodiments of the invention. Specifically, FIG. 2 is a side-view of the system 200 showing the cross section of the volume of soil 210 and the surrounding earth 215.

Step 105 can include measuring certain properties of the soil volume to determine baseline soil properties which can be used to inform subsequent steps of the remediation process, as further described herein. The measurements can be collected using measuring devices (not shown) and techniques that are known in the art, including, for example, the measurement of pH, oxidation-reduction potential (ORP), and the contaminant and biological environment using thin layer chromatography (TLC), high pressure liquid chromatography (HPLC), gas chromatography (GC), GC- mass spectroscopy (GC-MS), liquid chromatography - mass spectrometry (LC-MS/MS) Time of flight Ion Mass Spectrometry (TOF-SIMS), matrix assisted, laser desorption/ ionization - time of flight Mass Spectrometry (MALDI-ToF MS), gene amplification and genetic sequencing for genomics and metagenomics, next-gen sequencing of cDNA for transcriptomics, quantitative polymerase chain reaction (qPCR) and other methods. https://www.atsdr.cdc.gov/toxprofiles/tp69-c6.pdf, retrieved 12/5/18. Preferably, measurements taken from the soil include the concentration of at least BaP or other contaminants of interest. Other measured parameters can include moisture content, the size distribution of the soil, temperature of the soil and aeration level. It should be understood that one or more of the properties can be measured prior to, during or after remediation. Other measurements relating to the presence of contaminants in the environment, e.g., sediments, water, air, can also be taken in a similar manner as that performed with soil.

Step 105 can further include steps for preparing the soil 210 for remediation. Soil preparation can be performed using devices and techniques that are known in the art. The soil preparation can be performed as a function of the measured soil properties to, for instance, ensure that the soil properties are suitable for effective bioremediation of the soil. For instance, soil or sediment preparation can include screening the material to remove rocks and debris larger than a few inches, watering or drying the soils to provide optimum moisture conditions and adjusting the pH of the soil with acids or bases.

At step 110, the bioremediation bacteria *Geobacillus midousuji* is administered to the soil. More particularly, in one embodiment, ATCC Accession No. 55926 strain SH2B can be administered using techniques that are known in the art, e.g., spraying or mixing the bacteria with batches of the soil. Preferably an effective, degrading amount of the bacteria is administered to the soil. For example, the bacteria to soil ratio can range from about 0.001% to about 1% (w/w of wet weight of the *Geobacillus midousuji* to weight of the soil) and more preferably from about 0.01% to about 0.1% (w/w). Other ranges, include, 0.05-0.5% (w/w), 0. 02%-0.6% and 1%-5%.

The bacteria can be administered in an amount that is suitable for degrading the amount of BaP in the soil to an acceptable level as mandated by a government regulatory body. For example, the U.S. Environmental Protection Agency (EPA) defines regional screening level (RSL) for BaP in residential and industrial soil. In its Integrated Risk Information System, the U.S. EPA has published updated toxicity values for BaP stating that, at the time of publication, the updated RSL for residential and industrial soil, respectively, is 0.11 mg/kg (ppm) and 2.1 mg/kg (ppm). https://www.epa.gov/iris, retrieved 12/5/18. By way of further example, using the EPA IRIS site (https://www.epa.gov/risk/regional-screening-levels-rsls-generic-tables, accessed 2/15/2019), the table identifies, in the "Cancer" column for "residential" and "Composite worker soil," SLs of 1.8 mg/kg (ppm) and 22 mg/kg (ppm), respectively, for these populations using a THQ of 0.1. Using the RSL model the RSL is 1.78 mg/kg (ppm) for the residential soil and 66 mg/kg (ppm) for the construction soil.

Accordingly, the amount of bacteria administered can be a function of the level of BaP in the soil as well as a target level after remediation, e.g., more bacteria would be used for soils with 10 mg/kg (BaP weight/soil weight) than for soils with 2.1 mg/kg (BaP weight/soil weight). The amount of bacteria administered can also vary depending on the presence of other soil contaminant(s), the particular microbial culture(s) used to degrade said contaminant(s), the particular bioremediation approach, as well as other properties of the soil. In one embodiment, the amount of bacteria to be administered to the soil can range from about 0.01%, weight of the *Geobacillus midousuji* to weight of the soil, to more than 1%. The initial inoculate of bacteria also can be increased to accommodate greater concentrations of BaP in the soil or sediments. The preparation of the bacteria for soil treatments also can be varied. The bacteria may first be brought into logarithmic growth phase prior to its inoculation to the soil, or the bacteria can be applied to the soil while it is still in a dormant stage from storage.

In view of the present disclosure, determination of "an effective, degrading amount" as described herein is within the knowledge of one skilled in the art and various other methods can be used to determine the amounts of the bacteria required to effectively degrade the waste of interest. In the case of BaP and *Geobacillus midousuji,* the amount of BaP_can be determined using any technique known in the art, e.g., thin layer chromatography (TLC), high pressure liquid chromatography, gas chromatography (GC), GC- mass spectroscopy (GC-Mass Spec), Time of Flight Ion Mass Spectrometry (TOF-SIMS). The terms "effective" or "effective, degrading amount" can, in certain embodiments, refer to standard levels set by a governmental agency, e.g., US EPA.

Then, at step 115, the volume of soil to which the bioremediation bacteria have been added is incubated. Incubation of the bacteria, which facilitates the breakdown of BaP, can require certain environmental conditions. For example, it can be preferable to incubate *Geobacillus midousuji* bacterial strain ATCC Accession No. 55926 strain SH2B and/or ATCC 202050 strain SH2A at 40° to 70°C. Incubation and thus, the breakdown of BaP can be facilitated by maintaining preferable incubation conditions. Accordingly, step 115 can include at least partially enclosing the treatment area using, for example, a plastic liner or a temporary greenhouse-type enclosure 220, as shown in FIG. 2. Step 115 can also include controlling the ambient temperature within the enclosure such that the target temperature of the soil is maintained during the incubation period. Step 115 might also include the supply of air and/or oxygen and moisture to the environment within the enclosure 220 and, more generally, within and around the soil 210 using approaches that are known in the art and, as such, any such techniques can be used in connection with method 200.

Soil preparation can vary depending on the particular bioremediation approach, e.g., aerobic or anaerobic, low or high pH. Soil preparation can also vary depending on the type of soil contaminant(s) or the type of bacterial culture(s) used to degrade said contaminant(s).

The incubation can also be performed for an amount of time that is suitable for effective degradation of the contaminants by the applied bacteria. For example, *Geobacillus midousuji* bacterial strain ATCC Accession No. 55926 strain SH2B and/or ATCC 202050 strain SH2A can be incubated with the soil at a humidity ranging from about 80% to about 100% for a period of time ranging from about 1 hour to about 40 days. The prescribed amount of time can be predetermined based on parameters including the soil conditions, contaminant levels, the amount of bacteria applied, their degradation rates and the final contaminant levels desired and environmental conditions. The duration of the incubation can also be defined as a function of the estimated degradation rate for the contaminant(s) in question and confirmed by subsequent measurements of the remaining concentration of contaminants, e.g., BaP.

It should be further understood that one or more aspects of steps 105-115 can be repeated during incubation to effect further removal of the contaminants. Also, during incubation, aeration may be accomplished by periodically rotating the soil volume using techniques known in the art. In one embodiment, rotating can be performed once a day and can involve mixing the soil using an auger, a garden-tiller, a backhoe, shovel or other such soil processing equipment known in the art. Rotation also can be accomplished continually by incubating the material in a rotating chamber.

Incubation of the soil can require watering the soil regularly, in such a manner that the water content of the soil remains at an optimum level and therefore, degradation rate of the contaminants maintains at a maximum rate.

Furthermore, during incubation, the concentration of the bacteria and/or the soil contaminants and/or other measurable conditions of the soil or environment can be measured and monitored at least periodically, e.g., every 1, 2, 3, 4, 5, 10, 20, 30, 40, 50, 100 or 180 hours or more frequently as is needed. More preferably, the conditions can be monitored every 12, 24, 48, 96, or 192 hours. Thus, dynamic adjustments can be made during incubation based on the monitored conditions so as to facilitate the breakdown of the contaminants by the bacteria. For instance, additional amounts of the bacteria can be re-administered and distributed within the soil volume to adjust for process-loss and the pH of the soil can be maintaining in an optimum range.

The steps of the bioremediation method 200 can be implemented using a control system. The control system can be configured to actively monitor and dynamically control the operation of the bioremediation system 200 to improve operation and efficiency. For example, one or more of the aforementioned operational input parameters of the system and real-time conditions can be measured, monitored and analyzed using the control system before and/or during operation. Accordingly, the control system can define or adjust certain controllable system parameters so as to optimize the efficiency of the bioremediation system.

In addition, as shown in FIG. 1, the method 100 can further include, step 120, in which the soil is treated following incubation. Post-incubation treatment can be performed to enhance the degradation of the contaminants by the microbial population. Post-incubation treatment can also be performed to enhance degradation of any by-products of the bioremediation process. Post-incubation treatment such as heating above 100°C also can be used to destroy the remaining bacteria that were introduced initially.

In addition to the application of the *Geobacillus* strains to soil decontamination, this bioremediation is applicable to the decontamination of water and groundwater in which the contaminants are in dissolved, aqueous form. In this application, the bacteria and their associated enzymes are used to break-down water-miscible contaminants such as volatile organic compounds (chloroform, Bromodichloromethane, Dibromochloromethane) solvents, (Perchloroethene (PCE), Trichloroethene (TCE), 1,1,1-Trichloroethane (TCA), Chloromethane and Methylene chloride) gasoline hydrocarbons such as Benzene, Toluene, Ethylbenzene and Xylene (BTEX) and oxygenates and refrigerants. The *Geobacillus* treatment also is applicable to emerging contaminants in ground water such as 1,4-dioxane and per- and poly-fluorinated alkyl substances (PFAS).

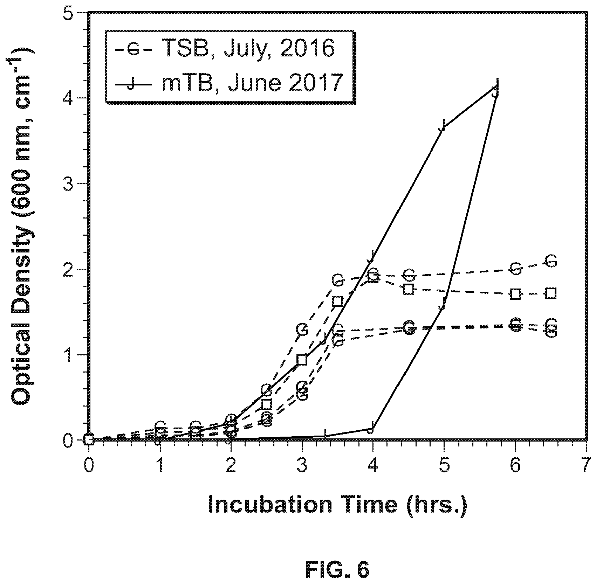

*Geobacillus midousuji* bacterial strain ATCC Accession No. 55926 strain SH2B and/or ATCC 202050 strain SH2A can be used with the methods of the present invention. In certain embodiments, the *Geobacillus* strain, e.g., SH2B (ATCC Accession No. 202050) may be modified or enhanced by pre-culture of the bacteria as described below (designated "enhanced bacterial strain") for improved growth, viability during storage, activity in environmental remediation and the production of useful products as follows. The initial isolate of the current strain used is *Geobacillus midousuji* bacterial strain ATCC Accession No. 202050 strain SH2B or, *Geobacillus midousuji* bacterial strain ATCC Accession No. 55926 strain SH2A. Growth enhancement of the *Geobacillus midousuji* bacterial strains was accomplished by optimizing media and growth conditions. In particular, the strains were found to grow better in a modified version of Terrific Broth (mTB) than in the Trypticase Soy Broth (TSB) that was specified in the original description of the strains. The mTB was further optimized to support the growth of G. *midousuji* by removing the glycerol component and adding a complement of trace metals to produce a Terrific Broth modified specifically to improve the titer of cells and their oxidases. Specifically, the new media formulation increased total cell yield from <2.0 × 10⁹ cells/ml to over 4 × 10⁹ cells/ ml, as shown in FIG. 6.

The viability of the cells during storage also was optimized through a series of strain selections in the laboratory. This was evidenced by the decrease in the lag period before the beginning of rapid growth from ~5 hrs. for unenhanced cells, to less than 3 hrs. for cell lines that were optimized for storage. In this way, the phenotype of the current working strain (SH2B2) has been improved by growth selection for cell production and storage by freezing at -80°C to be superior to the characteristics of the initial isolate available from ATCC. Table 1 below provides the components and amounts of an exemplary mTerrific Broth with Sucrose.

| **Component** | **Amount/ L** |
|---|---|
| Yeast extract | 24 g |
| Tryptone | 12 g |
| dipotassium phosphate (K₂HPO₄) | 9.7 g |
| monopotassium phosphate (KH₂PO₄) | 2.2 g |
| NaCl | 1 g |
| MnCl₂ * 4H₂O | 1 ml of 100 mM solution |
| FeSO₄ * 7H₂O | 1 ml of 1 mM solution |
| CaCl₂ * 2H₂O | 1 ml of 910 mM solution |
| MgSO₄ * 7H₂O | 1 ml of 590 mM solution |

FIG. 6 is a chart plotting optical density vs. incubation time and graphically illustrates the enhancement of strain cell yield by optimizing growth media from TSB to a modified Terrific Broth. Using the modified Terrific Broth (mTB), we isolated a strain that reaches a cell titer of over 4.0 × 10⁹ cell/ml and exhibits a lag time for the growth curves of approximately 3 hours.

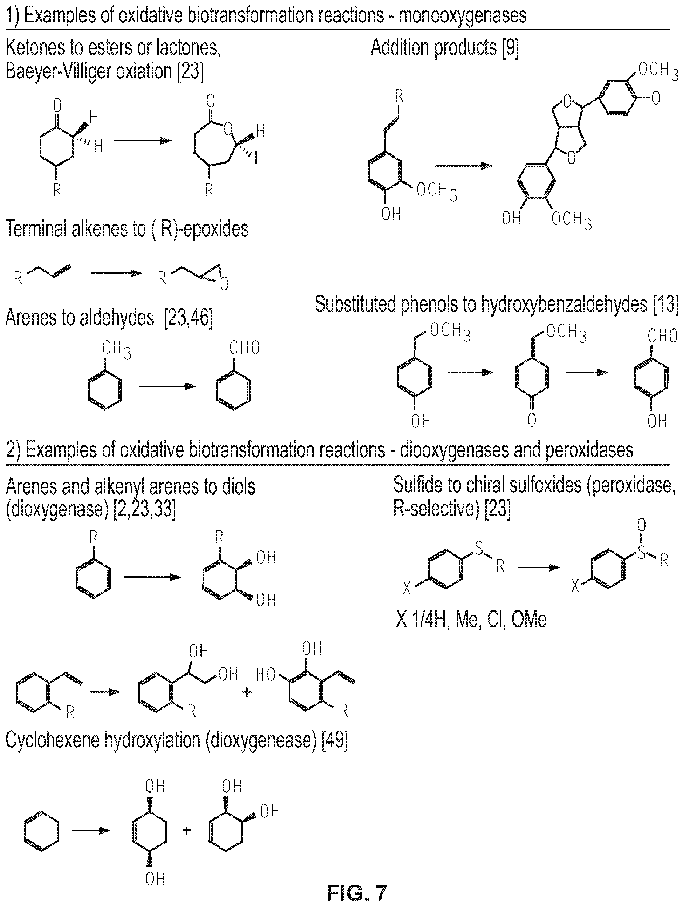

In view of the foregoing it can be appreciated that embodiments of the disclosed invention achieve various benefits including such as expanding the commercial application of strain *Geobacillus midousuji* ATCC Accession No. 55926 strain SH2B to the degradation of BaP and, 2) the production of intercellular and extracellular proteins. The production of extracellular proteins by *Geobacillus midousuji* bacterial strain ATCC Accession No. 55926 strain SH2B2 provides a basis for production of useful industrial enzymes for extracellular use. Total extracellular protein production at the end of the logarithmic growth phase of bacterial strain ATCC Accession No. 55926 strain SH2B2 ranged from 0.94 to 1.47 mg/ml, and was composed of over 20 distinct proteins as indicated by gel electrophoresis. These industrial applications include the use of enantiospecific reactions based on the dioxygenase and aconitase enzymes. FIG. 7 illustrates examples of oxidative biotransformation reactions for monooxygenases and examples of oxidative biotransformation reactions for dioxygenases and peroxidases.

The systems of the disclosure also include kits together with printed material containing instructions on use.

Embodiments of the invention disclosed herein further provide advantages in that they avoid the use of toxic chemicals for enantiospecific reactions and reactions involving non-polar solvents.

### I. BaP degradation

Testing results demonstrate that the *Geobacillus* strain described herein is capable of degrading Benzo[a]pyrene (BaP), a potent toxin produced by the incomplete combustion of organic matter (Figure 1 in supplemental material). This conclusion is based on an incubation of soil to which BaP was added to a concentration of -10 ppm and a mixture of polychlorinated biphenyls (PCBs) were added at a concentration of ~20 ppm. *Geobacillus midousuji* ATCC Accession No. 55926 strain SH2B was added to 3 separate incubation chambers containing 10 ml of soil. A fourth chamber was prepared as a control containing all of the same ingredients except the bacterium. The soil was incubated at 60°C for 5 days while being rotated. After 5 days, all of the treated incubations had significantly lower BaP and PCB levels than the control. While the *Geobacillus* strain previously was shown to degrade dioxins and polychlorinated biphenyls (PCBs), this is the first demonstration of its ability to degrade BaP. In another embodiment, the soil is not incubated at 60°C for 5 days and the *Geobacillus* strain is added to the soil which is maintained at ambient temperature, ranging from about 15° C to about 35°C.

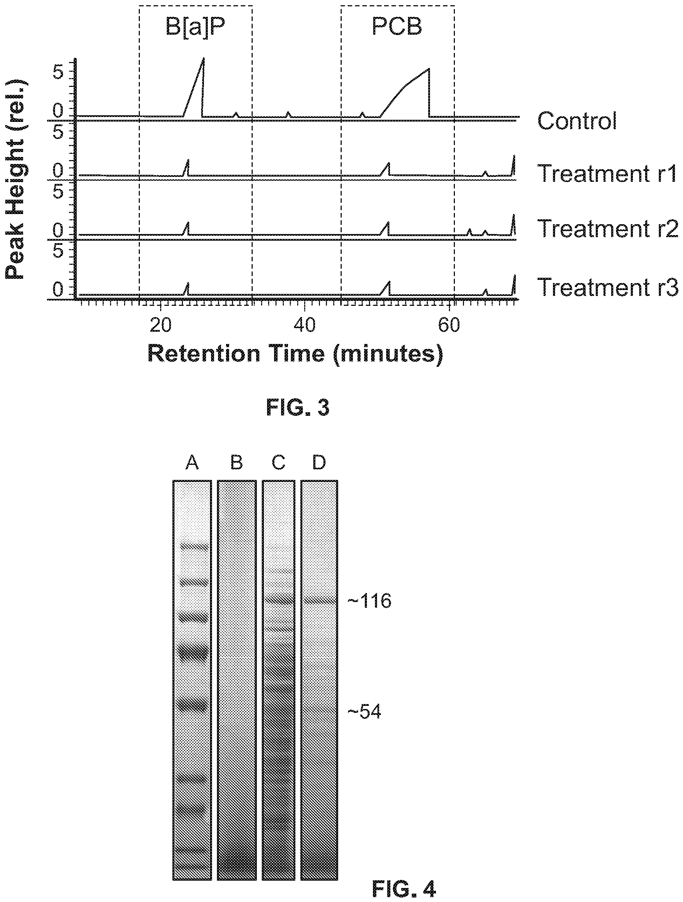

Results of degradation experiment with soils containing Benzo[a]pyrene (BaP) and PCBs are shown in FIG. 3. The graphic shows the gas chromatographic analysis of the extracted contaminants after 5 days of incubation at 62°C. 'Control' refers to the incubation without the *Geobacillus* strain. The three chromatograms marked 'Treatment' are triplicate analyses of the same sample incubated with the *Geobacillus* strain.

### II. Excreted protein products

### A. Number of peaks

A number of proteins appeared in the supernatant during the growth of the *Geobacillus* strain. Proteins were sampled in late logarithmic phase after growth on modified Terrific Broth. FIG. 4 illustrates the results of two-dimensional gel electrophoresis of supernatants from the bacterial broth. The leftmost lane 'A' shows protein size makers; lane 'B' shows fresh medium before growth; lane 'C' shows the supernatant from aerobic culture conditions; and lane 'D' denotes supernatant from anaerobic culture conditions. These results were generated consistently from samples taken from shake flask experiments as well as from stirred batch bioreactors. In either case, the supernatant from the centrifuged fermentation broth was concentrated with a dialysis membrane to ~20 times its original concentration. This concentrate was then loaded into the wells of polyacrylamide gels and its proteins separated by electrophoresis. The production of these proteins was significantly greater during aerobic growth than during anaerobic growth, but several proteins maintained significant bands in both growth conditions.

### B. Peak characterization

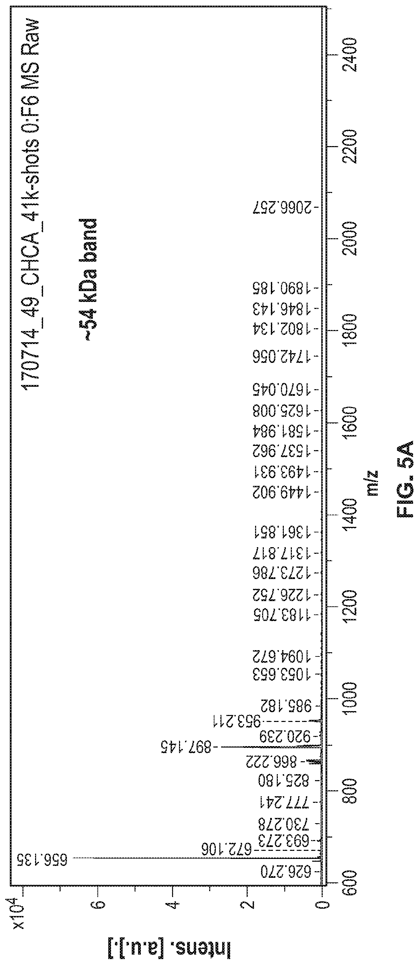

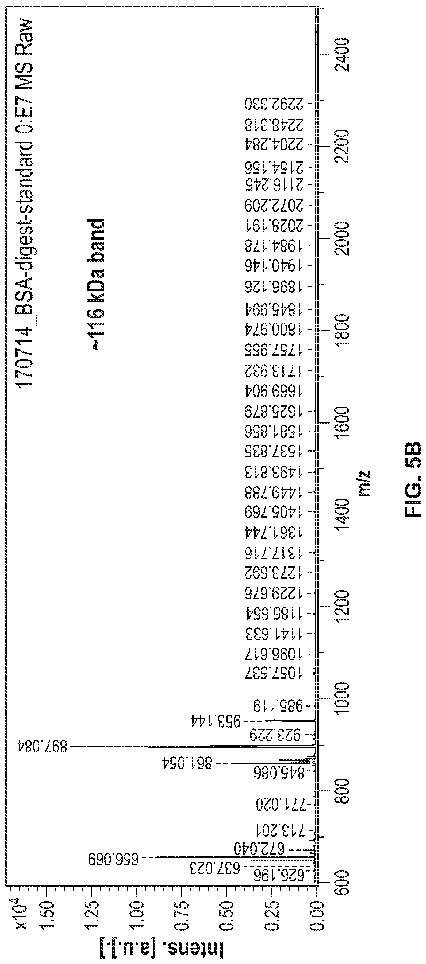

The 54 and 116 kDa protein bands were characterized further because they were the dominant bands that maintained their intensity in both aerobic and anaerobic growth. These bands were digested from the gel after electrophoresis and analyzed by Matrix-Assisted Laser Desorption/ Ionization - Time of Flight (MALDI-Tof) mass spectrometer analysis. Peptides that were recorded in both positive and negative beam modes were considered most significant, although some of the other peaks may represent valid peptides as well. Oligonucleotides may also appear on these scans, but matrices such as a-Cyano-4-hydroxycinnamic acid (CHCA) were used to minimize their peaks. FIGs. 5A and 5B illustrates the results of MALDI-Tof analysis of the two proteins. In both cases, samples were prepared with CHCA matrix (saturated) 7:3 water/ MeCN with 0.1% TFA and mixed with sample 7:3.

Two bacterial proteins from the current MALDI-ToF database were most similar to these peaks based on the peptide peaks they generated and are shown in Table 2, below. Previously sequenced proteins matching those analyzed from the *Geobacillus midousuji* bacterial strain ATCC Accession No. 55926 strain SH2B supernatant broth. Protein IDs were based on the closest matches of the peptide peaks detected by MALDI-ToF between the unknown proteins and those in the MALDI-ToF database.

| **Protein ID** | **Source organism** | **MW (kDa)** |
|---|---|---|
| 1-pyrroline-carboxylate dehydrogenase | *Geobacillus thermodenitrificans* | 56.5 |
| Aconitate hydratase | *Bacillus subtilis* | 99.3 |

### C. Commercial use of excreted proteins

The amount of enzymes secreted into the growth media and the relative ease of harvesting proteins from this matrix provides a basis for evaluating the commercial applications of this protein pool. The total amount of protein excreted by the Geobacillus strain is 0.94 to 1.47 mg/ml by the end of logarithmic growth. The aconitase peak is estimated to be approximately 10% of the total protein pool and the 1-pyrroline-carboxylate dehydrogenase peak to be approximately 5% of the total pool. On this basis, the production of aconitase and 1-pyrroline-carboxylate dehydrogenase during one of the optimized, batch fermentation runs would be approximately 0.12 and 0.06 mg/ml respectively.

### III. Dioxygenase capacity

The capacity of the *Geobacillus* strain to degrade ring-based organic contaminants has been attributed to its expression of the upper biphenyl pathway. The limiting step in this pathway is the first enzyme, a dioxygenase. The enzyme is denoted as a phenol- or toluene-dioxygenase based on the substrate used for its assay. It has been confirmed that the *Geobacillus midousuji* ATCC Accession No. 55926 strain SH2B expresses a toluene dioxygenase based on an enzyme specific assay as illustrated in FIG. 6. Specifically, FIG. 6 illustrates the results of an enzyme assay to test for the presence of the toluene dioxygenase enzyme in the *Geobacillus* strain. The build-up in indoxyl concentration (solid blue circles) indicates the presence of toluene dioxygenase acting on indole as a substrate. The open red squares reflect the subsequent buildup of indigo dye from indoxyl. The major enzyme responsible for the degradation of PCBs appears to be this dioxygenase.

The strains of the bacteria *Geobacillus midousuji* (*G. miduosuji*) and having ATCC accession No. 202050 or ATCC No. 55926 make a variety of chemically and biologically active molecules both inter- and extra-cellularly. These discoveries significantly extend the commercial applications of *G*. *midousuji* to enantiospecific reactions, surfactants and proteases, and the suppression of reactive oxygen species (ROS) in chemical and biological systems. Additional discoveries extend the types of organic pollutants which the SH2B strain can degrade to include poly-aromatic hydrocarbons (PaHs) such as the highly toxic benzo[a]pyrene, and emerging contaminants such as perfluorochemicals like perfluorooctanoic acid (PFOA) and 1,4-dioxane that currently lack cost effective treatments.

Bioproduct production and pollutant degradation were observed in laboratory culture experiments of several types. In one, the *G*. *midousuji* strains were incubated in a variety of liquid growth media including at various times and in various combinations, tryptone, peptone and yeast extract supplemented with various trace metals. Growth rates were optimum at about 62°C, although growth was recorded from 40-70°C, a temperature range that provides flexibility in the design of remediation applications. Growth rates were maximum with aeration, but growth was also confirmed using nitrate (NO₃⁻) instead of oxygen as the electron acceptor.

Bioproducts were analyzed in both the spent media and in cellular material after cell lysis by sonication. Cultures produced significant surfactants as evidenced by foaming of the media and the lowering of liquid surface tension. Proteinaceous material was analyzed by gel electrophoresis and matrix-assisted laser desorption/ ionization - time of flight (MALDI-ToF) mass spectrometry of individual protein bands. Several proteins were unique to aerobic growth and have applications in the suppression of ROS in chemical reaction systems and the biomedical field. The enzyme toluene 1,2 dioxygenase was demonstrated in the intercellular products by an enzyme assay. This Rieske non-heme iron oxygenase can be extracted and used in a cell-free mode for the degradation of aromatic ring structures in contaminant remediation and to carry out enantiospecific reactions for applications in green chemistry.

Other tests demonstrated that *G. midousuji* could degrade PCBs anaerobically. Growth conditions were as follows. Approximately 30 kg of soil contaminated with PCBs was mixed with *G. midousuji* and a solution of trypticase soy broth and 500 mM sodium nitrate and the mixture heated to 60°C for several weeks. This produced a significant decrease in PCBs and indicates that destruction of pollutants is possible without aerating the material, significantly reducing remediation costs.

Several successful incubation approaches were used. These included shake flask experiments with 150-ml and 300-ml liquid culture, stirred batch reactors with and without oxygen, 28-L fermentation reactors and packed-bed columns with live cells and glass beads. The packed-bed columns provide a way to remediation aqueous phase contaminants such as 1,4-dioxane in groundwater.

FIG. 8 - Contains photographs of 3 thin-layer chromatography (TLC) plates showing the analysis of extracts of the spent media from the Geobacillus SH2B strain. Plate A was stained with Phosphomolybdic Acid (PMA) and shows the presence of bands in the samples in lanes 2 and 3 that match the D3 and D4 bands from the di-rhamnolipid standard in lane 1. Plate B was stained with primuline and imaged under UV light and shows the presence of bands in the samples in lanes 3 and 4 that match the D3 and D4 bands from the di-rhamnolipid standard in lane 2. Plate C was stained with anthrone and shows the presence of bands in the samples in lanes 3 and 4 that match the D3 and D4 bands from the di-rhamnolipid standard in lane 2. Rhamnolipids are a class of biosurfactants which contain rhamnose as the sugar moiety linked to β-hydroxylated fatty acid chains. Rhamnolipids can be widely applied in many industries including petroleum, food, agriculture and bioremediation etc. Rhamnolipids are efficacious in bioremediation of organic and heavy metal polluted sites. They also facilitate degradation of waste hydrocarbons such as crude oil and vegetable oil.

As can be appreciated, the foregoing simulations and test results illustrate the benefits of the exemplary bioremediation systems and methods in accordance with embodiments of the present disclosure or invention.

The terminology used herein is for the purpose of describing particular embodiments only and is not intended to be limiting of the disclosure. As used herein, the singular forms "a", "an" and "the" are intended to include the plural forms as well, unless the context clearly indicates otherwise. It will be further understood that the terms "comprises" and/or "comprising", when used in this specification, specify the presence of stated features, integers, steps, operations, elements, and/or components, but do not preclude the presence or addition of one or more other features, integers, steps, operations, elements, components, and/or groups thereof.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

## Claims

1. A method for bioremediation of soil, sediment or wastewater containing benzo[a]pyrene (BaP), the method comprising the steps of:
administering live *Geobacillus midousuii,* or a mutant derived therefrom, to the soil, sediment or wastewater to be bioremediated; and,
incubating the *Geobacillus midousuii* in the soil, sediment or wastewater at a temperature ranging from about 40 to about 70°C and a humidity ranging from about 80% to about 100% for a period of time ranging from about 1 hour to about 20 days.

2. The method of claim 1, wherein the *Geobacillus midousuii* is strain SH2B (American Type Culture Collection (ATCC) No. 55926), strain SH2A (ATCC No. 202050), or mixtures thereof.

3. The method of claim 1, wherein the BaP concentration in the soil, sediment or wastewater after incubation is less than about 100 parts per million (PPM).

4. The method of claim 3, wherein the BaP concentration in the soil, sediment or wastewater after incubation is less than about 50 PPM.

5. The method of claim 4, wherein the BaP concentration in the soil, sediment or wastewater after incubation is less than about 20 PPM.

6. The method of claim 1, wherein the *Geobacillus midousuii* to the soil, sediment or wastewater ratio ranges from about 0.01% to about 1% w/w.

7. The method of claim 6, wherein the ratio ranges from about 0.01% to about 0.1% w/w.

8. The method of claim 6, wherein the ratio ranges from about 0.08% w/w to about 0.15% w/w.

9. The method of claim 8, wherein the ratio is about 0.1% w/w.

10. The method of claim 1, wherein the *Geobacillus midousuii* is activated into log phase growth before incubation with the soil, sediment or wastewater by incubation of the *Geobacillus midousuii* under an aerobic condition at a temperature ranging from about 60° to about 65° C in a medium comprising B -complex vitamin and amino-N with trace metals.

11. The method of claim 1, further comprising the step of rotating the soil, sediment or wastewater at least about 1 to about 5 times per day during incubation with the *Geobacillus midousuii.*

12. The method of claim 1, wherein the incubation of the *Geobacillus midousuii* in the soil, sediment or wastewater is done under aerobic conditions.

13. The method of claim 1, wherein the incubation of the *Geobacillus midousuii* in the soil, sediment or wastewater is done under anaerobic conditions.

14. The method of claim 1, further comprising purifying or harvesting at least one protein product.

## Patentansprüche

1. Verfahren zur Bioremediation von Boden, Sediment oder Abwasser, die Benzo[a]pyren (BaP) enthalten, wobei das Verfahren die folgenden Schritte umfasst:
Verabreichen von lebendem *Geobacillus midousuii* oder eines davon stammenden Mutanten an den Boden, das Sediment oder Abwasser, die der Bioremediation zu unterziehen sind; und
Inkubieren des *Geobacillus midousuii* in dem Boden, Sediment oder Abwasser bei einer Temperatur im Bereich von etwa 40 bis etwa 70 °C und einer Feuchtigkeit im Bereich von etwa 80 % bis etwa 100 % für eine Zeitdauer im Bereich von etwa 1 Stunde bis etwa 20 Tagen.

2. Verfahren nach Anspruch 1, wobei der *Geobacillus midousuii* Stamm SH2B (American Type Culture Collection (ATCC) Nr. 55926), Stamm SH2A (ATCC Nr. 202050) oder Mischungen davon ist.

3. Verfahren nach Anspruch 1, wobei die BaP-Konzentration in dem Boden, Sediment oder Abwasser nach Inkubation weniger als etwa 100 Parts per million (PPM) beträgt.

4. Verfahren nach Anspruch 3, wobei die BaP-Konzentration in dem Boden, Sediment oder Abwasser nach Inkubation weniger als etwa 50 PPM beträgt.

5. Verfahren nach Anspruch 4, wobei die BaP-Konzentration in dem Boden, Sediment oder Abwasser nach Inkubation weniger als etwa 20 PPM beträgt.

6. Verfahren nach Anspruch 1, wobei das Verhältnis von *Geobacillus midousuii* zu dem Boden, Sediment oder Abwasser im Bereich von etwa 0,01 m/m-% bis etwa 1 m/m-% liegt.

7. Verfahren nach Anspruch 6, wobei das Verhältnis im Bereich von etwa 0,01 m/m-% bis etwa 0,1 m/m-% liegt.

8. Verfahren nach Anspruch 6, wobei das Verhältnis im Bereich von etwa 0,08 m/m-% bis etwa 0,15 m/m-% liegt.

9. Verfahren nach Anspruch 8, wobei das Verhältnis etwa 0,1 m/m-% beträgt.

10. Verfahren nach Anspruch 1, wobei der *Geobacillus midousuii* vor Inkubation mit dem Boden, Sediment oder Abwasser durch Inkubation des *Geobacillus midousuii* unter einer aeroben Bedingung bei einer Temperatur im Bereich von etwa 60 °C bis etwa 65 °C in einem Medium, das B-Komplex-Vitamin und Amino-N mit Spurenmetallen umfasst, zu dem log-Phasen-Wachstum aktiviert wird.

11. Verfahren nach Anspruch 1, das weiter den Schritt des Rotierens des Bodens, Sediments oder Abwassers mindestens etwa 1- bis etwa 5-mal pro Tag während der Inkubation mit dem *Geobacillus midousuii* umfasst.

12. Verfahren nach Anspruch 1, wobei die Inkubation des *Geobacillus midousuii* in dem Boden, Sediment oder Abwasser unter aeroben Bedingungen erfolgt.

13. Verfahren nach Anspruch 1, wobei die Inkubation des *Geobacillus midousuii* in dem Boden, Sediment oder Abwasser unter anaeroben Bedingungen erfolgt.

14. Verfahren nach Anspruch 1, das weiter das Aufreinigen oder Ernten von mindestens einem Proteinprodukt umfasst.

## Revendications

1. Méthode de biorestauration du sol, de sédiments ou d'eaux usées contenant du benzo[a]pyrène (BaP), la méthode comprenant les étapes :
d'administration de *Geobacillus midousuii* vivant, ou d'un mutant dérivé de celui-ci, dans le sol, les sédiments ou les eaux usées à biorestaurer ; et
d'incubation de *Geobacillus midousuii* dans le sol, les sédiments ou les eaux usées à une température allant d'environ 40 à environ 70°C et à une humidité allant d'environ 80% à environ 100% pendant une période de temps allant d'environ 1 heure à environ 20 j ours.

2. Méthode selon la revendication 1, dans laquelle *Geobacillus midousuii* est la souche SH2B (American Type Culture Collection (ATCC) n° 55926), la souche SH2A (ATCC n° 202050), ou des mélanges de celles-ci.

3. Méthode selon la revendication 1, dans laquelle la concentration en BaP dans le sol, les sédiments ou les eaux usées après incubation est inférieure à environ 100 parties par million (ppm).

4. Méthode selon la revendication 3, dans laquelle la concentration en BaP dans le sol, les sédiments ou les eaux usées après incubation est inférieure à environ 50 ppm.

5. Méthode selon la revendication 4, dans laquelle la concentration en BaP dans le sol, les sédiments ou les eaux usées après incubation est inférieure à environ 20 ppm.

6. Méthode selon la revendication 1, dans laquelle le rapport entre *Geobacillus midousuii* et le sol, les sédiments ou les eaux usées va d'environ 0,01% à environ 1% p/p.

7. Méthode selon la revendication 6, dans laquelle le rapport va d'environ 0,01% à environ 0,1% p/p.

8. Méthode selon la revendication 6, dans laquelle le rapport va d'environ 0,08% p/p à environ 0,15% p/p.

9. Méthode selon la revendication 8, dans laquelle le rapport est d'environ 0,1% p/p.

10. Méthode selon la revendication 1, dans laquelle *Geobacillus midousuii* est activé en phase de croissance logarithmique avant l'incubation avec le sol, les sédiments ou les eaux usées par incubation de *Geobacillus midousuii* en conditions aérobies à une température allant d'environ 60°C à environ 65°C dans un milieu comprenant un complexe de vitamines B et de l'azote aminé avec des métaux en trace.

11. Méthode selon la revendication 1, comprenant en outre l'étape de retournement du sol, des sédiments ou des eaux usées au moins d'environ 1 à environ 5 fois par jour pendant l'incubation avec *Geobacillus midousuii.*

12. Méthode selon la revendication 1, dans laquelle l'incubation de *Geobacillus midousuii* dans le sol, les sédiments ou les eaux usées se fait dans des conditions aérobies.

13. Méthode selon la revendication 1, dans laquelle l'incubation de *Geobacillus midousuii* dans le sol, les sédiments ou les eaux usées se fait dans des conditions anaérobies.

14. Méthode selon la revendication 1, comprenant en outre la purification ou la récolte d'au moins un produit protéique.
